# EUROPEAN PATENT SPECIFICATION

(11) **EP 0 772 431 B1**
(45) Date of publication and mention of the grant of the patent: **27.08.2003**
(21) Application number: 95924237.1
(22) Date of filing: 16.06.1995
(51) Int. Cl.: A61K 7/16

(54) **ORAL HYGIENE COMPOSITION**
MUNDPLEGEMITTEL
COMPOSITION POUR L'HYGIENE BUCCO-DENTAIRE

(30) Priority: 16.06.1994 GB 9412048
(43) Date of publication of application: 14.05.1997
(73) Proprietor: The Boots Company PLC, Nottingham NG2 3AA (GB)
(72) Inventor: HAYWOOD, Julia, Nottingham NG2 3AA (GB); PULLEN, John Hamilton, Nottingham NG2 3AA (GB)
(74) Representative: Jones, Stephen Anthony
(86) International application number: EP9502308
(87) International publication number: WO95034275

(56) References cited:
- EP-A- 0 480 172
- DE-A- 1 444 311
- DE-A- 2 816 513
- DE-A- 2 922 671
- FR-A- 493 389
- FR-A- 2 625 676
- US-A- 3 146 170
- PATENT ABSTRACTS OF JAPAN vol. 4 no. 151 (C-28) [633] & JP,A,55 098111 (LION HAMIGAKI) & CHEMICAL ABSTRACTS, vol. 93, no. 22, 1 December 1980 Columbus, Ohio, US; abstract no. 210119s,
- CHEMICAL ABSTRACTS, vol. 99, no. 26, 26 December 1983 Columbus, Ohio, US; abstract no. 218412b, & TR. NAUCHNOIZSLED KHIM-FARM. INST., vol. 13, 1983 pages 171-178,
- CHEMICAL ABSTRACTS, vol. 98, no. 12, 21 March 1983 Columbus, Ohio, US; abstract no. 95486y, & TR. NAUCHNOIZSLED KHIM-FARM INST., vol. 12, 1982 pages 181-186,
- J Dent Res, vol 61, 11, 1982, pp 1236-1239

## Description

The present invention relates to oral hygiene compositions, especially toothwhitening compositions such as toothwhitening toothpastes. There is also provided inter alia the use of such compositions in the treatment and/or prevention of dental caries and/or gingivitis and in the cleansing of the buccal cavity for cosmetic purposes.

The term 'oral hygiene composition' as used herein denotes a formulation for use in any form of oral hygiene or dental treatment and includes inter alia dentifrices, mouthwashes, toothpastes, toothgels, toothpowders, lozenges and denture cleansing formulations and/or coatings for coating or impregnating dental accessories such as dental floss, toothbrush bristles and tooth picks.

The use of abrasive materials as cleansing and/or polishing agents in oral hygiene compositions is well known in the art.

For example GB-A-1186706 (Unilever) describes the use of particulate silica gel having an average particle diameter of from 2 to 20 microns as a polishing and cleansing agent in a dentifrice.

US-A-3538230 (Pader) describes the use of silica xerogels having an average particle size of from about 2 to about 20 microns as polishing and cleansing agents in a dentifrice.

However, use of high levels of such silica-type abrasives may risk causing damage to the teeth by wearing away the vital enamel layer and also dentine that may, for example, be exposed due to gum recession. There is thus a fine balance between achieving effective abrasive action to clean and/or whiten/polish the teeth on the one hand without causing damage on the other hand.

FR 2625676 (Pharmascience) discloses a toothpaste to eliminate dental plaque and the staining of teeth characterised in that it includes as abrasive cleaning agent, a mixture of mineral abrasive agents having particle sizes less than 20µm and an abrasive capacity less than 50, and a natural or synthetic organic polymer insoluble in water, having no gelling capacity and a particle size of between 50µm and 100µm. It is stated that the polymer is preferably a hydrophillic polymer, in particular cellulose or cellulose derivatives. The example given for such an organic polymer is the known product 'Avicel PH105' (RTM), which is a microcrystalline cellulose.

Microcrystalline cellulose is produced from grinding, cleaning and bleaching a source of cellulose (eg wood) and then hydrolysing the treated cellulose with acid (eg HCI), followed by spray drying. The result is a fractionated cellulose product with a high degree of crystallinity and a small amount of amorphous material. The treatment with acid produces a material which is partially hydrolysed with a low degree of polymerisation. Microcrystalline cellulose is to be contrasted with powdered cellulose, which is the material obtained simply after grinding, cleaning and bleaching the cellulose source. The absence of acid hydrolysis and spray drying in the production of powdered cellulose results in a less processed material of different particle shape, which comprises more amorphous material, has a higher degree of polymerisation and is cheaper to produce than microcrystalline cellulose.

The teaching of FR-2625676 as evidenced by the examples and the clearly envisages use of a microcrystalline cellulose ingredient of particle size 50-100µm as the organic polymer abrasive. This document also states that it is impossible to obtain advantageous results with the use of only a single abrasive agent.

US-A-5158764 (Degussa) describes a cellulose powder with a particle size of from 1 to 300 microns which may be used as a polishing agent in a dentifrice. The document gives examples of toothpaste formulations comprising a cellulose powder (particle size 40-150µm) in combination with a silica material available under the trade name "Aerosil" in an amount of up to 3% by weight. The silica is "pyrogenically produced" and so acts as a thickener rather than an abrasive. The document states that the cellulose material may be used to remove/eliminate plaque formations on the teeth.

Surprisingly, it has now been found in one aspect of the present invention, contrary to the teaching of the prior art, particulate cellulose may also be used together with a non-cellulose abrasive to remove stains from the teeth (as distinguished from plaque) and may be used to reduce and/or prevent the build-up of such stains.

The term "stain" as used herein means an extrinsic stain in the form of a coloured deposit on the teeth. Such stains may be caused, for example, by smoking, eating and drinking things such as tea, coffee and red wine, or by cationic antibacterials and chromogenic bacteria. Stain does not appear to have health threatening consequences and its removal is desirable for cosmetic reasons only. In contrast with stains, dental plaque is a complex mass of bacteria that forms on teeth and is the etiological factor responsible for caries and periodontal diseases. Plaque is soft and can generally be removed fairly easily, for example by brushing with water. Stains on the other hand are absorbed onto the hard pellicle on teeth and are much more difficult to remove.

A preferred cellulose cleaning/polishing agent comprises a high percentage of α-cellulose comprising 1,4-β-glycosidically linked D-glucose molecules with a degree of polymerisation of about 500 or more.

It has been found that combinations of a particulate cellulose cleansing/polishing agent of a smaller size (less than 50 µm) than previously known with various other abrasive agents provide surprisingly effective cleaning action without causing damage to tooth enamel.

According to the present invention broadly there is provided an oral hygiene composition comprising a combination of a particulate cellulose cleaning/polishing agent having a particle size of less than 50 µm together with a non-cellulose abrasive. In particular the cellulose particle size may be less than about 40 µm, for example about 35 µm.

The term "particulate cellulose cleaning/polishing agent" as used herein denotes a material which is capable of remaining substantially particulate in the oral hygiene composition on storage and in particular it is to be distinguished from cellulose gums and derivatives thereof. The term "powdered cellulose" as used herein denotes a material which is prepared as described above and in particular is to be distinguished from the more processed microcrystalline cellulose. Suitably the particulate cellulose may comprise the powdered and/or microcrystalline type. More suitably, the particulate cellulose is highly purified powdered cellulose such as that available under the trade names 'Elcema' from Degussa AG and 'Vitacel' from Allchem International. Suitably, the cellulose cleaning/polishing agent comprises from about 0.5% to about 25% by weight, preferably from about 1% to about 10%, suitably about 5% by weight of the oral hygiene composition.

Suitably the powdered cellulose and non-cellulose materials are present in a ratio from about 5:1 to 1:5 by weight.

Suitably, the non-cellulose abrasive is selected from: silica, alumina, insoluble metaphosphates, calcium carbonate, dicalcium phosphate (in dihydrate and anhydrous forms), calcium pyrophosphate, natural and synthetic clays, and particulate thermosetting polymerised resins selected from melamine-ureas, melamine-formaldehydes, urea-formaldehydes, melamine-urea-formaldehydes, cross-linked epoxides, melamines, phenolics, cross-linked polyesters and any compatible mixtures thereof.

Suitably, the composition comprises from about 0.1% to about 25% by weight, preferably from about 1% to about 15%, suitably about 5% by weight of the non-cellulose abrasive. Suitably, about 98% or more of the non-cellulose abrasive has a particle size of less than about 45 µm, optionally between about 25 µm and about 45 µm.

Preferably, the non-cellulose abrasive is a particulate silica abrasive. Suitable silica abrasives include the hydrated silicas, particularly those available under the trade names 'Sident' from Degussa AG, 'Zeodent' from J M Huber Corporation and 'Syloblanc' from W R Grace.

Unexpectedly, it has been found that certain alkali metal salts enhance the cleaning and/or polishing action of oral hygiene compositions, in particular those comprising a cellulose abrasive.

Therefore, in a further aspect of the present invention broadly there is provided an oral hygiene composition comprising a particulate cellulose cleaning/polishing agent and an agent to provide an enhanced cleaning/polishing action selected from one or more of: an alkali metal bicarbonate, an alkali metal orthophosphate, an alkali metal citrate and any compatible mixtures thereof. In such a composition the particulate cellulose abrasive may comprise powdered cellulose and/or microcrystalline cellulose. The cellulose abrasive may be the sole abrasive or be used in combination with other non-cellulose abrasives. Such compositions are suitable for removing stains and/or plaque. The preferred amounts and particle sizes of the particulate cellulose in such compositions are as given for the compositions described above.

Preferably, the composition further comprises an alkali metal bicarbonate, suitably sodium bicarbonate. Such a bicarbonate may be included in an amount of from about 0.1% to about 30%, suitably from about 1% to about 25%, more suitably from about 2% to about 10%, most suitably from about 2% to about 7% by weight of the oral hygiene composition, for example about 5% by weight. Inclusion of a bicarbonate provides an enhanced cleaning action.

Preferably, the composition further comprises an alkali metal orthophosphate, suitably monosodium phosphate, disodium phosphate and/or trisodium phosphate. Such phosphates may be included in an amount of from about 0.1% to about 10%, suitably from about 0.5% to about 5%, more suitably from about 0.5% to about 3% by weight, for example about 1% by weight of the oral hygiene composition. The presence of phosphates enhances cleaning action further.

It will be appreciated that where an alkali metal bicarbonate and an alkali metal orthophosphate are present together in the composition, the species selected must be compatible with one another. Strongly acidic species should not normally be present when bicarbonate is present, as otherwise an acid/base reaction may take place liberating carbon dioxide. It has been found that the combination of sodium bicarbonate, tripotassium citrate and trisodium orthophosphate is stable in use.

Preferably the composition further comprises an alkali metal citrate such as disodium citrate or tripotassium citrate. Such a salt gives a toothwhitening preparation of enhanced efficacy. Disodium citrate or tripotassium citrate are suitably used in amounts of about 0.1% to about 5%, preferably about 0.5% to about 3% by weight of the oral hygiene composition.

In yet a further aspect of the present invention broadly there is provided use of an agent to enhance the cleaning/polishing action of an oral hygiene composition, the agent being selected from: an alkali metal bicarbonate, an alkali metal orthophosphate, an alkali metal citrate and any compatible mixtures thereof.

The oral hygiene compositions described herein may be formulated for use in any form of interdental or periodontal treatment and may be in the form, for example, of a dentifrice or toothpowder.

Such compositions may, as appropriate, contain conventional materials such as, for example, humectants, surfactants, gelling agents, further abrasives, fluoride sources, desensitising agents, flavourings, colourings, sweeteners, preservatives, structuring agents, bactericides, anti-tartar agents and anti-plaque agents.

Suitable humectants for use in dentifrice compositions include polyhydric alcohols such as xylitol, sorbitol, glycerol, propylene glycol and polyethylene glycols. Mixtures of glycerol and sorbitol are particularly effective. A humectant helps to prevent dentifrice compositions from hardening on exposure to air, and may also provide a moist feel, smooth texture, flowability, and a desirable sweetness in the mouth. Suitably, such humectants may comprise up to about 85%, preferably up to about 60% by weight of the oral hygiene composition.

Suitable surfactants for use in dentifrices, mouthwashes etc. are usually water-soluble organic compounds, and may be anionic, nonionic, cationic or amphoteric species. The surfactant used should preferably be reasonably stable, and able to produce a foam in use.

Anionic surfactants include the water-soluble salts of C₁₀₋₁₈ alkyl sulphates (e.g. sodium lauryl sulphates), water soluble salts of C₁₀₋₁₈ ethoxylated alkyl sulphates, water soluble salts of C₁₀₋₁₈ alkyl sarcosinates, the water-soluble salts of sulfonated monoglycerides of C₁₀₋₁₈ fatty acids (e.g. sodium coconut monoglyceride sulfonates), alkyl aryl sulfonates (e.g. sodium dodecyl benzene sulfonate) and sodium salts of the coconut fatty acid amide of N-methyltaurine.

Nonionic surfactants suitable for use in oral compositions include the products of the condensation of alkylene oxide groups with aliphatic or alkylaromatic species, and may be for example, polyethylene oxide condensates of alkyl phenols, ethylene oxide/propylene oxide copolymers (available from BASF Wyandotte Chemical Corporation under the trade name 'Pluronic'), ethylene oxide/ethylene diamine copolymers, ethylene oxide condensates of aliphatic alcohols, long chain tertiary amine oxides, long chain tertiary phosphine oxides, long chain dialkyl sulfoxides and mixtures thereof. Alternatives include ethoxylated sorbitan esters such as those available from ICI under the trade name "Tween".

Cationic surfactants are generally quaternary ammonium compounds having one C₈₋₁₈ alkyl chain and include, for example, lauryl trimethylammonium chloride, cetyl trimethylammonium bromide, cetyl pyridinium chloride, di-isobutylphenoxyethoxyethyldimethylbenzylammonium chloride, coconut alkyltrimethylammonium nitrite and cetyl pyridinium fluoride. Also useful are benzyl ammonium chloride, benzyl dimethyl stearylammonium chloride, and tertiary amines having one C₁₋₁₈ hydrocarbon group and two (poly)oxyethylene groups.

Amphoteric surfactants are generally aliphatic secondary and tertiary amines comprising aliphatic species which may be branched or unbranched, and in which one of the aliphatic species is a C₈₋₁₈ species and the other contains an anionic hydrophillic group, for example, sulfonate, carboxylate, sulphate, phosphonate or phosphate. Examples of quaternary ammonium compounds are the quaternized imidazole derivatives available under the trade name 'Miranol' from the Miranol Chemical Company.

Suitably, the surfactant is included in an amount up to about 20%, preferably up to about 10% by weight of the oral hygiene composition.

Structuring agents may be required in, for example, dentifrices and gums to provide desirable textural properties and "mouthfeel". Suitable agents include natural gum binders such as gum tragacanth, xanthan gum, gum karaya and gum arabic, seaweed derivatives such as Irish moss and alginates, smectite clays such as bentonite or hectorite, carboxyvinyl polymers and water-soluble cellulose derivatives such as hydroxyethyl cellulose and sodium carboxymethyl cellulose. Improved texture may also be achieved, for example, by including colloidal magnesium aluminium silicate. Suitably, the structuring agent is included in an amount from up to about 5%, preferably up to about 3% by weight of the oral hygiene composition.

Fluoride sources suitable for use in all oral hygiene compositions of the present invention include sodium fluoride, zinc fluoride, potassium fluoride, aluminium fluoride, lithium fluoride, sodium monofluorophosphate, stannous fluoride, ammonium fluoride, ammonium bifluoride and amine fluoride.

Preferably, the fluoride source is present in an amount sufficient to provide from about 50 ppm to about 4,000 ppm fluoride ions in the composition. Inclusion of a fluoride source is beneficial, since fluoride ions are known to become incorporated into the hydroxyapatite of tooth enamel, thereby increasing the resistance of the enamel to decay. Fluoride is also now thought to act locally on the tooth enamel, altering the remineralisation-demineralisation balance in favour of remineralisation. Inclusion of a fluoride source is also desirable when a polyphosphate anti-calculus agent is included, in order to inhibit the enzymic hydrolysis of such polyphosphates by salivary phosphatase enzymes.

Suitable desensitising agents include, for example, formaldehyde, potassium nitrate, tripotassium citrate, potassium chloride and strontium chloride (suitably as hexahydrate), strontium acetate (suitably as hemihydrate) and sodium citrate.

Flavouring agents may be added to increase palatability and may include, for example, menthol, oils of peppermint, spearmint, wintergreen, sassafras and clove. Sweetening agents may also be used, and these include D-tryptophan, saccharin, dextrose, aspartame, levulose, acesulfam, dihydrochalcones and sodium cyclamate. Typically, such flavouring agents are included in amounts up to about 5%, preferably up to about 2% by weight of the oral hygiene composition. Colouring agents and pigments may be added to improve the visual appeal of the composition. Suitable colourants include dyes, such as FD & C blue No.1, D & C yellow No.10 and D & C yellow No.3. A suitable and commonly used pigment is pigment grade titanium dioxide, which provides a strong white colour.

Suitably, as described above, the compositions of the invention may include a further antimicrobial agent as a preservative and/or anti-plaque agent. Suitable antimicrobial agents include zinc salts (such as zinc citrate), cetyl pyridinium chloride, the bis-biguanides (such as chlorhexidine), aliphatic amines, bromochloro-phene, hexachlorophene, salicylanilides, quaternary ammonium compounds and triclosan. Enzymic systems providing a source of a natural biocide may be used as alternatives to or in combination with the biocides listed. For example, a system comprising lactoperoxidase and glucose oxidase may be used to generate antimicrobial amounts of hydrogen peroxide in the presence of glucose, water and oxygen.

The composition may also comprise an anti-calculus agent. Suitable anti-calculus agents include zinc salts such as zinc citrate and zinc chloride and poly-phosphates. Suitable pyrophosphates include the sodium and potassium pyrophosphates, preferably disodium pyrophosphate, dipotassium pyrophosphate, tetrasodium pyrophosphate and tetrapotassium pyrophosphate. A preferred source of pyrophosphate is a mixture of tetrasodium pyrophosphate and tetrapotassium pyrophosphate.

It will be appreciated that when choosing components from the lists above, the components chosen must be chemically and physically compatible with one another.

Particularly suitable oral compositions are those in the form of a toothpaste or liquid dentifrice. A liquid dentifrice contains components commonly associated with a paste eg abrasives, humectants and actives, but the viscosity is considerably lower, and is preferably below about 50,000 cps.

According to another aspect of the present invention, there is provided a method of cleaning the buccal cavity for cosmetic purposes by oral application of any oral hygiene composition as defined above. As used herein the term "buccal cavity" denotes inter alia the mouth, the teeth and the gums.

In a further aspect, the present invention provides a method of treating and/or preventing dental caries and/or gingivitis by oral application of any oral hygiene composition as defined above.

In yet a further aspect of the present invention, there is provided the use of a composition as defined above in the treatment and/or prevention of dental caries and/or gingivitis.

In a still further aspect of the present invention, there is provided the use of a composition as defined above in the manufacture of a medicament for the treatment and/or prevention of dental caries and/or gingivitis.

The nature of the present invention is illustrated by the following Examples, which should not be considered to limit the scope of the present invention in any way.

### Example 1

A toothwhitening toothpaste was prepared to the following composition:

| Component | Concentration (% w/w) |
|---|---|
| Non-crystalline sorbitol (70% in aqueous | 50 |
| solution) | |
| Silica thickener (available under the trade | |
| name "Sident 22S") | 10 |
| Silica abrasive (available under the trade | |
| name "Sident 9") | 8 |
| Cellulose abrasive (available from | |
| Degussa AG under the trade name | |
| "Elcema P050") | 2 |
| Sodium lauryl sulphate, (available under | |
| the trade name "Empicol LZ PDR") | 1.5 |
| Sodium carboxymethylcellulose | 0.9 |
| Sodium fluoride BP | 0.33 |
| Titanium dioxide PH EUR | 0.75 |
| Sodium saccharin BP | 0.26 |
| Flavour | 1 (by volume) |
| Sodium citrate BP | 2.5 |
| Purified water BP | 22.7 |

### Method of Formulation

The sodium fluoride, sodium saccharin, sodium citrate were dissolved in purified water and the sorbitol solution was added. The silica abrasive, silica thickener, cellulose abrasive, sodium carboxymethylcellulose and titanium dioxide were added to the above mixture and stirred under vacuum until the mixture was homogeneous. Then the sodium lauryl sulphate and flavour ingredient were added to this mixture which was stirred again under vacuum until homogeneous. The resultant toothpaste showed excellent cleaning properties in use.

### Example 2

A toothwhitening toothpaste was prepared to the following composition:

| Component | Concentration (% w/w) |
|---|---|
| Non-crystalline sorbitol (70% in aqueous solution) | 50 |
| Silica thickener (available under the trade name "Sident 22S") | 10 |
| Cellulose abrasive (available from Degussa AG under the trade name "Elcema P050") | 5 |
| Silica abrasive (available under the trade name "Sident 9") | 5 |
| Sodium lauryl sulphate (available under the trade name "Empicol LZ PDR") | 1.5 |
| Sodium carboxymethylcellulose | 0.9 |
| Sodium fluoride BP | 0.33 |
| Titanium dioxide PH EUR | 0.75 |
| Sodium saccharin BP | 0.26 |
| Flavour | 1 (by volume) |
| Sodium citrate BP | 2.5 |
| Monosodium phosphate BP | 0.5 |
| Disodium phosphate BP | 0.5 |
| Purified water BP | qs |

### Method of Formulation

The sodium fluoride, sodium saccharin, sodium citrate, monosodium phosphate and disodium phosphate were dissolved in purified water and the sorbitol solution was added. The silica abrasive, silica thickener, cellulose abrasive, sodium carboxymethylcellulose and titanium dioxide were added to the above mixture, which was stirred under vacuum until it was homogeneous. Then the sodium lauryl sulphate and flavour ingredient were added to the mixture which was stirred again under vacuum until homogeneous. The resultant toothpaste showed excellent cleaning properties in use.

### Example 3

| Component | Concentration (% w/w) |
|---|---|
| Non-crystalline sorbitol (70% in aqueous solution) | 50.00 |
| Silica thickener (available under the trade name "Sident 22S") | 10.00 |
| Cellulose abrasive (available from Degussa under the trade name "Elcema P050") | 5.00 |
| Silica abrasive (available under the trade name "Sident 9") | 5.00 |
| Sodium lauryl sulphate (available under the trade name "Empicol LZ PDR") | 1.50 |
| Sodium carboxymethylcellulose | 0.90 |
| Sodium fluoride BP | 0.33 |
| Titanium dioxide PH EUR | 0.75 |
| Sodium saccharin BP | 0.26 |
| Flavour | 1.00 (By volume) |
| Sodium citrate BP | 2.50 |
| Sodium bicarbonate BP | 5.00 |
| Purified water BP | qs |

### Method of Formulation

The sodium fluoride, sodium saccharin, sodium citrate, sodium bicarbonate were dissolved in purified water and the sorbitol solution was added. The silica abrasive, silica thickener, cellulose abrasive, sodium carboxymethylcellulose and titanium dioxide were added to the above mixture, which was stirred under vacuum until homogeneous. Then the sodium lauryl sulphate and flavour ingredient were added to the mixture, which was stirred again under vacuum until homogeneous. The resultant toothpaste showed excellent cleaning properties in use.

### Example 4

| Component | Concentration (% w/w) |
|---|---|
| Non-crystalline sorbitol (70% in solution) | 45 |
| Silica thickener (available under the trade name "Sident 22S") | 9.5 |
| Cellulose abrasive (from Degussa under the trade name "Elcema P050") | 5 |
| Silica abrasive (available under the trade name "Sident 9") | 5 |
| Sodium lauryl sulphate (available under the trade name "Empicol LZ PDR") | 1.5 |
| Sodium carboxymethylcellulose | 0.7 |
| Sodium fluoride BP | 0.33 |
| Titanium dioxide PH EUR | 0.75 |
| Sodium saccharin BP | 0.26 |
| Tripotassium citrate BP | 2.5 |
| Potassium nitrate | 5 |
| Anhydrous trisodium phosphate | 2 |
| Toothpaste flavour | 1 |
| Purified water BP | 21.46 |

### Method of formulation

The sodium fluoride, sodium saccharin, tripotassium citrate, potassium nitrate and trisodium phosphate were dissolved in water which was heated to 45°C and the sorbitol solution was added. The silica thickener, silica abrasive, cellulose abrasive, titanium dioxide, sodium carboxymethyl cellulose and sodium lauryl sulphate were mixed together and added to the aqueous phase under vacuum. The resultant mixture was stirred under vacuum until the sodium carboxymethyl cellulose was fully hydrated and the mixture was homogenous. The flavour ingredient was added and the mixture stirred again under vacuum until homogenous. The resultant toothpaste showed excellent cleaning properties in use.

### Example 5

| Component | Concentration (% w/w) |
|---|---|
| Non-crystalline sorbitol (70% in aqueous solution) | 45 |
| Silica thickener (available under the tradename "Sident 22S") | 7.5 |
| Cellulose abrasive (from Degussa under the trade name "Elcema P050") | 5 |
| Silica abrasive (available under the trade name "Sident 8") | 5 |
| Silica abrasive (available under the trade name "Sident 10") | 2 |
| Sodium lauryl sulphate (available under the trade name "Empicol LZ PDR") | 1.5 |
| Sodium carboxymethylcellulose | 0.6 |
| Sodium fluoride BP | 0.24 |
| Sodium saccharin BP crystalline | 0.26 |
| Titanium dioxide PH EUR | 0.75 |
| Tetrapotassium pyrophosphate | 2.77 |
| Tetrasodium pyrophosphate | 2.77 |
| EDTA tetrasodium salt | 0.2 |
| Toothpaste flavour | 1 |
| Monosodium phosphate | 0.50 |
| Disodium phosphate | 0.50 |
| Purified water | 24.41 |

### Method of Formulation

The sodium fluoride, sodium saccharin, tetrapotassium pyrophosphate, tetrasodium pyrophosphate, EDTA tetrasodium salt, monosodium phosphate and disodium phosphate were dissolved in water and the sorbitol solution was added. The silica thickener, silica abrasives, cellulose abrasive, sodium carboxymethyl cellulose, titanium dioxide and sodium lauryl sulphate were mixed together and added to the above aqueous phase under vacuum. The resultant mixture was stirred under vacuum until sodium carboxymethyl cellulose was fully hydrated and the mixture was homogeneous. The flavour ingredient was added and the mixture was stirred again until homogeneous. The resultant toothpaste showed excellent cleaning properties in use.

### Example 6

| Component | Concentration (% w/w) |
|---|---|
| Non-crystalline sorbitol (70% in solution) | 45 |
| D-panthenol (pro-vitamin B5 - a soothing agent) | 0.15 |
| Hexetidine 99 (antibacterial agent) | 0.1 |
| Cellulose abrasive (available from Degussa under the trade name "Elcema P050") | 12 |
| Titanium Dioxide PH EUR | 0.9 |
| Hydroxycellulose (a gelling agent available under the trade name "Natrasol 250 G") | 4 |
| Sodium monofluorophosphate | 0.84 |
| Sodium saccharin | 0.26 |
| Allantoin BCP'34 (a soothing agent) | 0.15 |
| Cocoamido propyl betain (a surfactant) | 4.5 |
| Toothpaste flavour | 1 (by volume) |
| Colour | 0.001 |
| Purified Water BP | 31.099 |

### Method of formulation

The sodium monoflurophosphate, sodium saccharin, allantoin and D-panthenol were dissolved in purified water and the sorbitol solution was added. The cellulose abrasive, titanium dioxide and the hydroxycellulose were mixed together and added to the aqueous phase under vacuum. The resultant mixture was stirred under vacuum until hydroxycellulose was fully hydrated and the mixture was homogeneous. The colour, flavour, hexetidine and cocoamido propyl betain ingredients were added to the mixture, which was stirred again under vacuum until homogeneous. The resultant toothpaste showed excellent cleaning properties in use.

### Example 7

| Component | Concentration (% w/w) |
|---|---|
| Non-crystalline sorbitol (70% in aqueous solution) | 45 |
| Sodium saccharin BP crystalline | 0.26 |
| Sodium monofluorophospahte | 0.84 |
| Sodium carboxymethylcellulose | 0.85 |
| Silica abrasive (available under the trade name "Zeodent 163") | 12.22 |
| Silica abrasive (available under the trade name "Zeodent 113") | 6 |
| Cellulose abrasive (available from Degussa under the trade name "Elcema P0500") | 4 |
| Zinc citrate trihydrate | 0.5 |
| Titanium dioxide PH EUR | 0.75 |
| Sodium lauryl sulphate | 1.75 |
| Sodium bicarbonate | 2 |
| Bromochlorophene | 0.1 |
| Toothpaste flavour | 0.939 |
| Purified water | 25.041 |

### Method of Formulation

The sodium monofluorophoshpate, sodium saccharin and sodium bicarbonate were dissolved in water and the sorbitol solution was added. The toothpaste silica thickener, toothpaste silica abrasive, cellulose abrasive, titanium dioxide, zinc citrate, trihydrate, sodium lauryl sulphate and sodium carboxymethylcellulose were mixed together and added to the aqueous phase under vacuum. The resultant mixture was stirred under vacuum until the sodium carboxymethylcellulose had fully hydrated and the mixture was homogeneous. The bromochlorophene was dissolved in the flavour ingredient and added to the previous mixture which was then stirred again until homogeneous. The resultant toothpaste showed excellent cleaning properties in use.

## Claims

1. An oral hygiene composition comprising a non-microcrystalline, powdered, particulate cellulose cleaning / polishing agent having a particle size of less than 50 µm, together with a non-cellulose abrasive present in an amount sufficient to remove, reduce and/or prevent build up of stain on teeth.

2. An oral hygiene composition as claimed in claim 1, in which the cellulose has a particle size of less than about 40 µm.

3. An oral hygiene composition as claimed in claim 1, in which the non-cellulose abrasive comprises silica.

4. An oral hygiene composition as claimed in any preceding claim in which the non-cellulose abrasive has a particle size of between about 25 µm and about 45 µm.

5. An oral hygiene composition as claimed in any preceding claim, which further comprises an agent to provide enhanced cleaning action selected from: alkali metal bicarbonate, alkali metal orthophosphate, alkali metal citrate and any compatible mixtures thereof.

6. An oral hygiene composition as claimed in claim 5, in which the agent to provide enhanced cleaning action is selected from: sodium bicarbonate, trisodium orthophosphate and tripotassium citrate.

7. The use of an oral hygiene composition comprising a non-microcrystalline, powdered, particulate cellulose cleaning / polishing agent having a particle size of less than 50 µm, together with a non-cellulose abrasive in the manufacture of a medicament to remove, reduce and/or prevent build-up of stains on teeth.

8. The use as claimed in claim 7, in which the cellulose has a particle size of less than about 40 µm.

9. The use as claimed in claim 7 in which the non-cellulose abrasive comprises silica.

10. The use as claimed in any of claims 7 to 9 in which the non-cellulose abrasive has a particle size of between about 25 µm and about 45 µm.

11. The use as claimed in any of claims 7 to 10, which further comprises an agent to provide enhanced cleaning action selected from: alkali metal bicarbonate, alkali metal orthophosphate, alkali metal citrate and any compatible mixtures thereof.

12. The use as claimed in claim 11, in which the agent to provide enhanced cleaning action is selected from: sodium bicarbonate, trisodium orthophosphate and tripotassium citrate.

## Patentansprüche

1. Mundhygienezusammensetzung, umfassend ein nichtmikrokristallines, pulvriges, partikuläres Cellulosereinigungs-/poliermittel mit einer Partikelgröße von weniger als 50 µm, sowie ein Nicht-Cellulose-Abrasivmittel, das in einer Menge vorliegt, die ausreicht, um eine Fleckenbildung auf Zähnen zu entfernen, reduzieren und/oder zu verhindern.

2. Mundhygienezusammensetzung nach Anspruch 1, bei der die Cellulose eine Partikelgröße von weniger als etwa 40 µm hat.

3. Mundhygienezusammensetzung nach Anspruch 1, bei der das Nicht-Cellulose-Abrasivmittel Siliciumdioxid umfasst.

4. Mundhygienezusammensetzung nach einem der vorherigen Ansprüche, bei der das Nicht-Cellulose-Abrasivmittel eine Partikelgröße zwischen etwa 25 µm und etwa 45 µm hat.

5. Mundhygienezusammensetzung nach einem der vorherigen Ansprüche, die ferner ein Mittel zur Bereitstellung einer besseren Reinigungswirkung umfasst, ausgewählt aus: Alkalimetallbicarbonat, Alkalimetallorthophosphat, Alkalimetallcitrat und jedem beliebigen kompatiblen Gemisch davon.

6. Mundhygienezusammensetzung nach Anspruch 5, wobei das Mittel zur Erzielung einer besseren Reinigungswirkung ausgewählt ist aus: Natriumbicarbonat, Trinatriumorthophosphat und Trikaliumcitrat.

7. Verwendung einer Mundhygienezusammensetzung, umfassend ein nichtmikrokristallines, pulvriges, partikuläres Cellulosereinigungs-/poliermittel mit einer Partikelgröße von weniger als 50 µm, sowie ein Nicht-Cellulose-Abrasivmittel zur Herstellung eines Medikamentes zum Entfernen, Reduzieren und/oder Verhindern einer Fleckenbildung auf Zähnen.

8. Verwendung nach Anspruch 7, wobei die Cellulose eine Partikelgröße von weniger als etwa 40 µm hat.

9. Verwendung nach Anspruch 7, wobei das Nicht-Cellulose-Abrasivmittel Siliciumdioxid umfasst.

10. Verwendung nach einem der Ansprüche 7 bis 9, wobei das Nicht-Cellulose-Abrasivmittel eine Partikelgröße zwischen etwa 25 µm und etwa 45 µm hat.

11. Verwendung nach einem der Ansprüche 7 bis 10, ferner umfassend ein Mittel zur Bereitstellung einer besseren Reinigungswirkung, ausgewählt aus: Alkalimetallbicarbonat, Alkalimetallorthophosphat, Alkalimetallcitrat und beliebigen kompatiblen Gemischen davon.

12. Verwendung nach Anspruch 11, wobei das Mittel zur Bereitstellung einer besseren Reinigungswirkung ausgewählt ist aus: Natriumbicarbonat, Trinatriumorthophosphat und Trikaliumcitrat.

## Revendications

1. Composition d'hygiène orale comprenant un agent nettoyant / polisseur pulvérisé non microcristallin à cellulose particulaire offrant une taille de particules inférieure à 50 µm, ainsi qu'un agent abrasif non cellulosique présent en une quantité suffisante pour éliminer, réduire et/ou empêcher l'accumulation de taches sur les dents.

2. Composition d'hygiène orale selon la revendication 1, où la cellulose se compose de particules de taille inférieure à environ 40 µm.

3. Composition d'hygiène orale selon la revendication 1, où l'agent abrasif non cellulosique comprend de la silice.

4. Composition d'hygiène orale selon l'une quelconque des revendications précédentes, où l'agent abrasif non cellulosique se compose de particules d'une taille située entre environ 25 µm et environ 45 µm.

5. Composition d'hygiène orale selon l'une quelconque des revendications précédentes, qui comporte en outre un agent assurant une action nettoyante renforcée sélectionné à partir de : bicarbonate de métal alcalin, orthophosphate de métal alcalin, citrate de métal alcalin et de tout mélange compatible de ceux-ci.

6. Composition d'hygiène orale selon la revendication 5, où l'agent assurant une action nettoyante renforcée sélectionné à partir de : bicarbonate de sodium, d'orthophosphate trisodique et de citrate tripotassique.

7. Usage d'une composition d'hygiène orale comprenant un agent nettoyant / polisseur pulvérisé non microcristallin à cellulose particulaire offrant une taille de particules inférieure à 50 µm, ainsi qu'un agent abrasif non cellulosique dans la fabrication d'un médicament visant à éliminer, réduire et/ou empêcher l'accumulation de taches sur les dents.

8. Usage selon la revendication 7, où la cellulose présente des particules de taille inférieure à environ 40 µm.

9. Usage selon la revendication 7, où l'agent abrasif non cellulosique comporte de la silice.

10. Usage tel que revendiqué dans l'une quelconque des revendications 7 à 9, où l'agent abrasif non cellulosique présente des particules de taille située entre environ 25 µm et environ 45 µm.

11. Usage tel que revendiqué dans l'une quelconque des revendications 7 à 10, qui en outre comporte un agent destiné à assurer une action nettoyante renforcée sélectionné à partir de bicarbonate de métal alcalin, orthophosphate de métal alcalin, citrate de métal alcalin et de tout mélange compatible de ceux-ci.

12. Usage tel que revendiqué dans la revendication 11, où l'agent destiné à assurer une action nettoyante renforcée est sélectionné à partir de bicarbonate de sodium, d'orthophosphate trisodique et de citrate tripotassique.
